# EUROPEAN PATENT SPECIFICATION

(11) **EP 0 975 682 B1**
(45) Date of publication and mention of the grant of the patent: **19.05.2004**
(21) Application number: 98906654.3
(22) Date of filing: 24.02.1998
(51) Int. Cl.: C08F 10/00, C08F 4/70, C07F 15/04, C07F 15/02, C07F 15/06, C07F 15/00, C07D 233/96, C07D 285/10, C07F 9/6584

(54) **GROUP 8-10 TRANSITION METAL OLEFIN POLYMERIZATION CATALYSTS**
GRUPPE 8-10 ÜBERGANGSMETALLKATALYSATOR FÜR DIE POLYMERISATION VON OLEFINEN
CATALYSEURS DE POLYMERISATION D'OLEFINES DE METAL DE TRANSITION DU GROUPE 8-10

(30) Priority: 18.04.1997 US 44691 P; 01.05.1997 US 45333 P; 02.05.1997 US 45355 P
(43) Date of publication of application: 02.02.2000
(73) Proprietor: EASTMAN CHEMICAL COMPANY, Kingsport, TN 37660 (US)
(72) Inventor: MACKENZIE, Peter, Borden, Kingsport, TN 37660 (US); KILLIAN, Christopher, Moore, Gray, TN 37615 (US); MCDEVITT, Jason, Patrick, Wake Forest, NC 27587 (US); MOODY, Leslie, Shane, Johnson City, TN 37604 (US)
(74) Representative: Evans, David Charles
(86) International application number: PCT/US1998/003512
(87) International publication number: WO 1998/047933

(56) References cited:
- JOHNSON L K ET AL: "NEW PD(II)- AND NI(II)-BASED CATALYSTS FOR POLYMERIZATION OF ETHYLENE AND ALPHA-OLEFINS" JOURNAL OF THE AMERICAN CHEMICAL SOCIETY, vol. 117, no. 23, 14 June 1995, page 6414/6415 XP002025459 cited in the application
- PETTINARI C ET AL: "Tin(IV) and organotin(IV) complexes containing mono or bidentate N -donor ligands II. 4-Phenylimidazole derivatives. Crystal and molecular structure of [bis(4-phenylimidazole) trimethyltin(IV)] chloride" JOURNAL OF ORGANOMETALLIC CHEMISTRY, vol. 515, no. 1, 31 May 1996, page 119-130 XP004035998

## Description

The present invention is directed to olefin polymers, such as polyethylene, polypropylene, and copolymers of ethylene and propylene, and to the preparation of such olefin polymers. These polymers include homopolymers of olefins, as well as copolymers of different olefins. The present invention is also directed to the processes of making these olefin polymers, which typically use a transition metal complex, in which the transition metal is a Group 8-10 metal, as the polymerization catalyst. The polymers have a wide variety of applications, including use as packaging materials and adhesives. In addition, the present invention is directed to catalysts for the polymerization of olefins.

### Background of the Invention

Olefin polymers are used in a wide variety of products, from sheathing for wire and cable to film. Olefin polymers are used, for instance, in injection or compression molding applications, in extruded films or sheeting, as extrusion coatings on paper, for example photographic paper and digital recording paper, Improvements in catatysts have made it possible to better control polymerization processes, and, thus, influence the properties of the bulk material. Increasingly, efforts are being made to tune the physical properties of plastics for lightness, strength, resistance to corrosion, permeability, and optical properties, for particular uses. Chain length, polymer branching and functionality have a significant impact on the physical properties of the polymer. Accordingly, catalysts are constantly being sought in attempts to obtain a catalytic process which permits more efficient and better controlled polymerization of olefins.

Conventional polyolefins are prepared by a variety of polymerization techniques, including homogeneous and heterogeneous polymerizations. Certain transition metal catalysts, such as those based on titanium compounds (e.g. TiCl₃ or TiCl₄) in combination with organoaluminum cocatalysts, are used to make linear and linear low density polyethylenes as well as poly-α-olefins such as polypropylene. These so-called "Ziegler-Natta" catalysts are quite sensitive to oxygen and are ineffective for the copolymerization of nonpolar and polar monomers.

Recent advances in non-Ziegler-Natta olefin polymerization catalysis include the following:
L. K Johnson et al., WO Patent Application 96/23010, disclose the polymerization of olefins using cationic nickel, palladium, iron, and cobalt complexes containing diimine and bisoxazaline ligands. This document also describes the polymerization of ethylene, acyclic olefins, and/or selected cyclic olefins and optionally selected unsaturated acids or esters such as acrylic acid or alkyl acrylates to provide olefin homopolymers or copolymers;
EP-A-381,495 describes the polymerization of olefins using palladium and nickel catalysts which contain selected bidentate phosphorous containing ligands;
L. K Johnson et al., *J. Am. Chem. Soc.* **1995**, *117*, 6414, describe the polymerization of olefins such as ethylene, propylene, and 1-hexene using cationic α-diimine-based nickel and palladium complexes. These catalysts have been described to polymerize ethylene to high molecular weight branched polyethylene. In addition to ethylene, Pd complexes act as catalysts for the polymerization and copolymerization of olefins and methyl acrylate.
G.F. Schmidt et al., *J. Am. Chem. Soc.* **1985**, *107*, 1443, describe a cobalt(III) cyclopentadienyl catalytic system having the structure [C₅Me₅(L)CoCH₂CH₂-µ-H]⁺, which provides for the "living" polymerization of ethylene.
M. Brookhart et al., *Macromolecules* **1995,** *28*, 5378, disclose using such "living" catalysts in the synthesis of end-functionalized polyethylene homopolymers;
U. Klabunde, U. S. Patent Nos. 4,906,754, 4,716,205, 5,030,606, and 5,175,326, describes the conversion of ethylene to polyethylene using anionic phosphorous/oxygen donors ligated to Ni(II). The polymerization reactions were run between 25 and 100°C with modest yields, producing linear polyethylene having a weight-average molecular weight ranging between 8K and 350 K In addition, Klabunde describes the preparation of copolymers of ethylene and functional group containing monomers;
M. Peuckert et al., *Organomet*. **1983**, *2*(5), 594, disclose the oligomerization of ethylene using phosphine/carboxylate donors ligated to Ni(II), which showed modest catalytic activity (0.14 to 1.83 TO/s). The oligomerizations were carried out at 60 to 95° C and 1x10⁵ to 8x10⁵ Pas (10 to 80 bar) ethylene in toluene, to produce linear α-olefins;

R.E. Murray, U.S. Patents Nos. 4,689,437 and 4,716,138, describes the oligomerization of ethylene using phosphine/sulfonate donors ligated to Ni(II). These complexes show catalyst activities approximately 15 times greater than those reported with phosphine/carboxylate analogs.

W. Keim et al., *Angew. Chem. Int. Ed. Eng*. **1981**, *20*, 116, and V.M. Mohring, et al., *Angew. Chem. Int. Ed. Eng*. **1985**, *24*,1001, disclose the polymerization of ethylene and the oligomerization of α-olefins with aminobis(imino)phosphorane nickel catalysts; G. Wilke, *Angew. Chem. Int. Ed. Engl*. **1988**, *27*, 185, describes a nickel allyl phosphine complex for the polymerization of ethylene.

K.A.O. Starzewski et al., *Angew. Chem. Int. Ed. Engl.* **1987**, *26*, 63, and U. S. Patent 4,691,036, describe a series of bis(ylide) nickel complexes, used to polymerize ethylene to provide high molecular weight linear polyethylene.

WO Patent Application 97/02298 discloses the polymerization of olefins using a variety of neutral N, O, P, or S donor ligands, in combination with a nickel(0) compound and an acid.

Brown et al., WO 97/17380, describes the use of Pd α-diimine catalysts for the polymerization of olefins including ethylene in the presence of air and moisture.

Fink et al., U. S. Patent 4,724,273. have described the polymerization of α-olefins using aminobis(imino)phosphorane nickel catalysts and the compositions of the resulting poly(α-olefins).

Additional recent developments are described by Sugimura et al., in JP96-84344, JP96-84343, and WO 9738024, and by Yorisue et al., in JP96-70332.

Notwithstanding these advances in non-Ziegler-Natta catalysis, there remains a need for efficient and effective Group 8-10 transition metal catalysts for effecting polymerization of olefins. In addition, there is a need for novel methods of polymerizing olefins employing such effective Group 8-10 transition metal catalysts. In particular, there remains a need for Group 8-10 transition metal olefin polymerization catalysts with both improved temperature stability and functional group compatibility. Further, there remains a need for a method of polymerizing olefins utilizing effective Group 8-10 transition metal catalysts in combination with a Lewis acid so as to obtain a catalyst that is more active and more selective.

### SUMMARY OF THE INVENTION

According to the present invention there are provided compounds, processes and compositions as set forth in the claims hereinafter. The present invention provides a batch or continuous process for the polymerization of olefins, comprising: contacting, at a temperature from -100°C to 200°C, one or more monomers of the formula RCH=CHR³ with (i) a compound of the formula II, or tautomers thereof, (ii) a suitable precursor selected from zerovalent Ni, Pd, Co, or Fe or compounds thereof, and, optionally, (iii) a neutral Lewis acid; 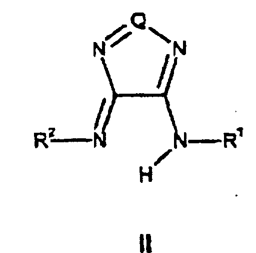 wherein R and R³ are each, independently, hydrogen, hydrocarbyl or fluoroalkyl and may be linked to form a cyclic olefin;
R¹ and R² are each independently hydrocarbyl, substituted hydrocarbyl, or silyl;
Q is (i) C-R⁴, wherein R⁴ is hydrocarbyl, substituted hydrocarbyl, heteroatom connected hydrocarbyl, or heteroatom connected substituted hydrocarbyl, (ii) P(NH₂)₂, or (iii) S(NH)(NH₂) or S(O)(OH).

This invention also provides a batch or continuous process for the polymerization of olefins, comprising: contacting, at a temperature from -100 °C to 200 °C, one or more monomers of the formula RCH=CHR³, with (i) an anionic compound of the formula III, (ii) a suitable divalent metal precursor selected from Ni(II), Pd(II), Co(II), or Fe(II) compounds, and, optionally, (iii) a neutral Lewis acid; 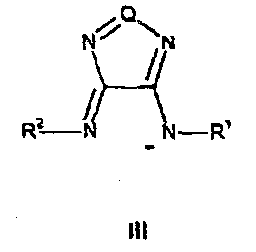 wherein R and R³ are each, independently, hydrogen, hydrocarbyl or fluoroalkyl and may be linked to form a cyclic olefin;
R¹ and R² are each independently hydrocarbyl, substituted hydrocarbyl, or silyl;
Q is C-R⁴, where R⁴ is hydrocarbyl, substituted hydrocarbyl, heteroatom connected hydrocarbyl, heteroatom connected substituted hydrocarbyl, or O-Si(tert-butyl)(CH₃)₂.

This invention also provides a batch or continuous process for the production of polyolefins, comprising: contacting, at a temperature from -100 °C to 200 °C, one or more monomers of the formula RCH=CHR³ with (i) a transition metal complex of formula I, and, optionally, (ii) a Lewis acid; 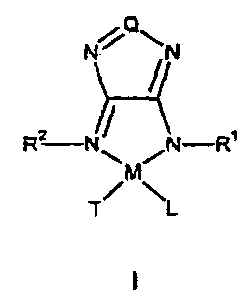 wherein R and R³ are each, independently, hydrogen, hydrocarbyl or substituted hydrocarbyl and preferably such as fluoroalkyl, may be linked to form a cyclic olefin;
R¹ and R² are each independently hydrocarbyl, substituted hydrocarbyl, or silyl;
Q is (i) C-R⁴, where R⁴ is hydrocarbyl, substituted hydrocarbyl, heteroatom connected hydrocarbyl, or heteroatom connected substituted hydrocarbyl, (ii) P(NH₂)₂, or (iii) S(NH)(NH₂) or S(O)(OH);
L is a monoolefin or a neutral Lewis base that can be displaced by a monoolefin;
T is hydrogen, hydrocarbyl or substituted hydrocarbyl, or may be taken together with L to form a π-allyl group; and
M is Ni(II), Pd(II), Co(II) or Fe(II).

This invention also provides a compound of formula **I**: 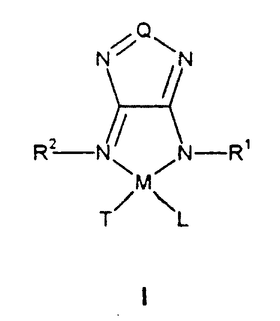 wherein R¹ and R² are each independently hydrocarbyl, substituted hydrocarbyl, or silyl;
Q is (i) C-R⁴, where R⁴ is hydrocarbyl, substituted hydrocarbyl, heteroatom connected hydrocarbyl, or heteroatom connected substituted hydrocarbyl, (ii) P(NH₂)₂, or (iii) S(NH)(NH₂) or S(O)(OH);
L is a monoolefin or a neutral Lewis base that can be displaced by a monoolefin;
T is hydrogen, hydrocarbyl or substituted hydrocarbyl, or may be taken together with L to form a π-allyl group; and
M is Ni(II), Pd(II), Co(II) or Fe(II).

This invention also provides a compound of formula **II**. 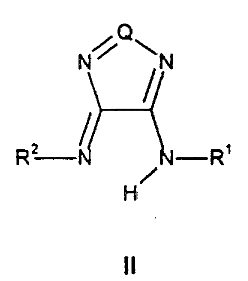 wherein R¹ and R² are both sterically hindered aryl rings;
Q is (i) C-R⁴, where R⁴ is hydrocarbyl, substituted hydrocarbyl, heteroatom connected hydrocarbyl, or heteroatom connected substituted hydrocarbyl, (ii) P(NH₂)₂, or (iii) S(NH)(NH₂) or S(O)(OH).

This invention also provides a batch or continuous process for the polymerization of olefins, comprising: contacting one or more monomers of the formula RCH=CHR³ with a binucleating or multinucleating ligand complexed to a Group 8-10 transition metal M and one or more Lewis acids, wherein the Lewis acid or acids are bound to one or more heteroatoms which are π-conjugated to the donor atom or atoms bound to the transition metal M; and wherein R and R³ each, independently, represent a hydrogen, a hydrocarbyl, a fluoroalkyl, or may be linked to form a cyclic olefin.

This invention also provides a composition comprising (i) a Group 8-10 transition metal M, (ii) one or more Lewis acids, and (iii) a binucleating or multinucleating compound of the formula II: 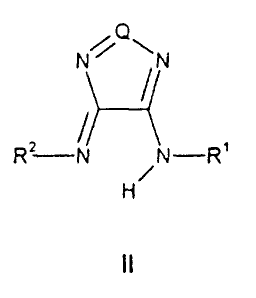 wherein the Lewis acid or acids are bound to one or more heteroatoms which are π-conjugated to the donor atom or atoms bound to the transition metal M;
R¹ and R² are both sterically hindered aryl rings;
Q is (i) C-R⁴, where R⁴ is hydrocarbyl, substituted hydrocarbyl, heteroatom connected hydrocarbyl, or heteroatom connected substituted hydrocarbyl, (ii) P(NH₂)₂, or (iii) S(NH)(NH₂) or S(O)(OH).

### Detailed Description of the Invention

In this disclosure certain chemical groups or compounds are described by certain terms and symbols. These terms are defined as follows:

Symbols ordinarily used to denote elements in the Periodic Table take their ordinary meaning, unless otherwise specified.

Examples of neutral Lewis bases include (i) ethers, for example, diethyl ether or tetrahydrofuran, (ii) organic nitriles, for example acetonitrile, (iii) organic sulfides, for example dimethylsulfide, or (iv) monoolefins, for example, ethylene, hexene or cyclopentene.

Examples of neutral Lewis acids include B(C₆F₅)₃, Al(CH₃)₃, methylaluminoxane, BPh₃, B(3,5-(CF₃)C₆H₃)₃.

A "sterically hindered aryl" means (i) a phenyl ring with hydrocarbyl, substituted hydrocarbyl, F, Cl, Br or silyl substituents at both the 2- and 6-positions, optionally substituted elsewhere with hydrocarbyl, substituted hydrocarbyl, F, Cl, Br, silyl, hydroxy, methoxy, nitro, cyano, phenylsulfonyl, CO₂Me, CO₂H, C(O)CH₃, CF₃, or fluoroalkyl substituents, (ii) a 2-substituted napth-1-yl ring, optionally substituted elsewhere with hydrocarbyl, substituted hydrocarbyl, F, Cl, Br, silyl, hydroxy, methoxy, nitro, cyano, phenylsulfonyl, CO₂Me, CO₂H, C(O)CH₃, CF₃, or fluoroalkyl substituents, (iii) an 9-anthracenyl ring, optionally substituted elsewhere with hydrocarbyl, substituted hydrocarbyl, F, Cl, Br, silyl, hydroxy, methoxy, nitro, cyano, phenylsulfonyl, CO₂Me, CO₂H, C(O)CH₃, CF₃, or fluoroalkyl substituents, or (iv) an aromatic substituted hydrocarbyl with steric properties functionally equivalent (in the context of this invention) to one or more of the following sterically hindered aryls: 2,6-dimethylphenyl, 2,4,6-trimethylphenyl, 2,6-diisopropylphenyl, 2,6-dimethyl-4-nitrophenyl, 2,6-dimethyl-4-phenylsulfonylphenyl, 2-isopropyl-6-methylphenyl, 2,6-bis(trifluoromethyl)phenyl, 2,6-dimethyl-4-methoxyphenyl, 2-methylnapth-1-yl, 9-anthracenyl, 2,6-diclorophenyl, 2,6-dibromophenyl, 2-tert-butyl-6-methylphenyl, 2-trimethylsilylnapth-1-yl, 2-chloro-6-methylphenyl, 4-cyano-2,6-dimethylphenyl, 2,6-diisopropyl-4-methoxyphenyl, 2,4,6-tri-tert-butylphenyl, and 2-chloro-6-tert-butylphenyl.

A "hydrocarbyl" group means a linear, branched or cyclic group which contains only carbon and hydrogen atoms. Examples include: C₁-C₂₀ alkyl; C₁-C₂₀ alkyl substituted with one or more groups selected from C₁-C₂₀ alkyl, C₃-C₈ cycloalkyl or aryl; C₃-C₈ cycloalkyl; C₃-C₈ cycloalkyl substituted with one or more groups selected from C₁-C₂₀ alkyl, C₃-C₈ cycloalkyl or aryl; C₆-C₁₄ aryl; and C₆-C₁₄ aryl substituted with one or more groups selected from C₁-C₂₀ alkyl, C₃-C₈ cycloalkyl or aryl; where the term "aryl" preferably denotes a phenyl, napthyl, or anthracenyl group.

Examples of groups useful as the group Q include (i) C-aryl, where aryl is phenyl, pentafluorophenyl, or phenyl substituted with F, Cl, Br, methoxy, cyano, nitro, CO₂H, CF₃, fluoroalkyl, or phenylsulfonyl, (ii) C-tert-butyl, C-CF₃, or C-fluoroalkyl, (iii) C-S-R⁵ or C-O-R⁵, where R⁵ is C₁-C₂₀ alkyl, silyl, phenyl, or phenyl substituted with F, Cl, Br, CF₃, fluoroalkyl, CO₂H, methoxy, cyano, nitro, or phenylsulfonyl, (iv) C-silyl, (v) P(NH₂)₂, or (vi) S(NH)(NH₂) or S(O)(OH).

A "silyl" group refers to a SiR⁶₃ group where Si is silicon and R⁶ is hydrocarbyl or substituted hydrocarbyl or silyl, as in Si(SiR⁶₃)₃.

A "heteroatom" refers to an atom other than carbon or hydrogen. Preferred heteroatoms include oxygen, nitrogen, phosphorus, sulfur, selenium, silicon, arsenic, chlorine, bromine, and fluorine.

A "fluoroalkyl" as used herein refers to a C₁-C₂₀ alkyl group substituted by one or more fluorine atoms.

A "substituted hydrocarbyl" refers to a hydrocarbyl substituted with one or more heteroatoms. Examples include: 2,6-dimethyl-4-methoxyphenyl, 2,6-diisopropyl-4-methoxyphenyl, 4-cyano-2,6-dimethylphenyl, 2,6-dimethyl-4-nitrophenyl, 2,6-difluorophenyl, 2,6-dibromophenyl, 2,6-dichlorophenyl, 4-methoxycarbonyl-2,6-dimethylphenyl, 2-tert-butyl-6-chlorophenyl, 2,6-dimethyl-4-phenylsulfonylphenyl, 2,6-dimethyl-4-trifluoromethylphenyl, 2,6-dimethyl-4-hydroxyphenyl, 9-hydroxyanthr-10-yl, 2-chloronapth-1-yl, 4-methoxyphenyl, 4-nitrophenyl, 9-nitroanthr-10-yl, CH₂OCH₃, cyano, trifluoromethyl, or fluoroalkyl.

A "heteroatom connected hydrocarbyl" refers to a group of the type Z-(hydrocarbyl), where Z is a divalent heteroatom, preferably O or S, or Z-(hydrocarbyl)₂, where Z is a trivalent heteroatom, preferably N. Examples include: OCH₃, OPh, N(CH₃)₂, SCH₃, or SPh.

A "heteroatom connected substituted hydrocarbyl" refers to a group of the type Z-(substituted hydrocarbyl), where Z is a divalent heteroatom, preferably O or S, or Z-(substituted hydrocarbyl)₂, where Z is a trivalent heteroatom, preferably N. Examples include: OCH₂CF₃, SC(O)CH₃, and 1-morpholinyl.

A "mono-olefin" refers to a hydrocarbon containing one carbon-carbon double bond.

A "suitable precursor" refers to a zerovalent or divalent transition metal compound which may be combined with compound II, and optionally a neutral Lewis acid, to form an active olefin polymerization catalyst. Examples of suitable precursors include: bis(1,5-cycloctadiene)nickel(0) and bis[(1, 2, 3-η³-2-propenyl)nickel(II)].

A "suitable divalent metal precursor" refers to a divalent transition metal compound which may be combined with compound **III**, and optionally a neutral Lewis acid, to form an active olefin polymerization catalyst. Examples include: (1,2-dimethoxyethane)nickel(II) dibromide, bis[(µ-chloro)(1, 2, 3-η³-2-propenyl)nickel(II)], bis[(µ-chloro)(1, 2, 3-η³-2-propenyl)palladium(II)], bis[(µ-chloro)(1, 2, 3-η³-1-trimethylsilyloxy-2-propenyl)nickel(II)], CoBr₂, FeBr₂.

A "π-allyl" group refers to a monoanionic group with three sp² carbon atoms bound to a metal center in a η³-fashion. Any of the three sp² carbon atoms may be substituted with a hydrocarbyl, substituted hydrocarbyl, heteroatom connected hydrocarbyl, heteroatom connected substituted hydrocarbyl, or O-silyl group. Examples of π-allyl groups include:

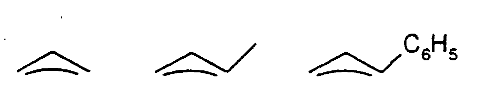

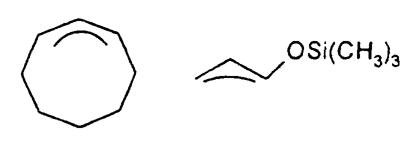

The term "polymer" as used herein is meant a species comprised of monomer units and having a degree of polymerization (DP) of ten or higher.

As used herein, the terms "monomer" or "olefin monomer" refer to the olefin or other monomer compound before it has been polymerized; the term "monomer units" refers to the moieties of a polymer that correspond to the monomers after they have been polymerized.

Described herein is a process for the polymerization of olefins. Preferred olefins include ethylene and α-olefins such as propylene, 1-butene, 1-hexene, 1-octene, and cyclic olefins such as cyclopentene.

When the polymerizations are conducted in the liquid phase, said liquid phase may include solvent or neat monomer. The molar ratio of neutral Lewis acid to transition metal complex can be from 0 to 10000, preferably 0 to 100, more preferably 0 to 10. The pressure at which the ethylene polymerizations and copolymerizations take place can be from 101.3 kPa to 101.3 MPa (from 1 atmosphere to 1000 atmospheres), preferably 101.3 kPa to 10.1 MPa (1 to 100 atmospheres).

While not wishing to be bound by theory, the present inventors believe that the Lewis acid may be acting to further activate the catalysts disclosed herein via coordination to one or more of those heteroatoms which are not directly bound to the transition metal M, but which are π-conjugated to the nitrogens which are bound to the transition metal M. Substituents which contain additional Lewis basic groups, including, but not limited to, methoxy groups, positioned so as to further promote the binding of the Lewis acid at such π-conjugated heteroatoms, are also included in this invention. An example of secondary Lewis acid binding would include the following: 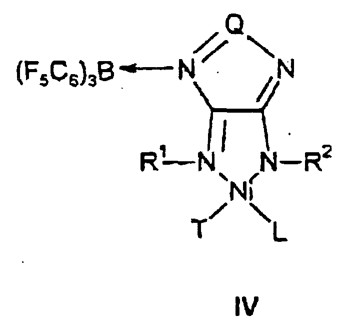 wherein R¹ and R² are each independently hydrocarbyl, substituted hydrocarbyl, or silyl;
Q is (i) C-R⁴, where R⁴ is hydrocarbyl, substituted hydrocarbyl, heteroatom connected hydrocarbyl, or heteroatom connected substituted hydrocarbyl, (ii) P(NH₂)₂, or (iii) S(NH)(NH₂) or S(O)(OH);
L is a monoolefin or a neutral Lewis base that can be displaced by a monoolefin; and
T is hydrogen, hydrocarbyl or substituted hydrocarbyl, or may be taken together with L to form a π-allyl group.

The polymerizations may be conducted as solution polymerizations, as non-solvent slurry type polymerizations, as slurry polymerizations using one or more of the olefins or other solvent as the polymerization medium, or in the gas phase. One of ordinary skill in the art, with the present disclosure, would understand that the catalyst could be supported using a suitable catalyst support and methods known in the art. Substantially inert solvents, such as toluene, hydrocarbons, may be used. Propylene and 1-butene are excellent monomers for use in slurry-type copolymerizations and unused monomer can be flashed off and reused.

Polymerization temperature and pressure have significant effects on copolymer structure, composition, and molecular weight. Suitable polymerization temperatures are preferably from -100 °C to 200 °C, more preferably in the 20 °C to 150 °C range.

After the reaction has proceeded for a time sufficient to produce the desired polymers, the polymer can be recovered from the reaction mixture by routine methods of isolation and/or purification.

High molecular weight resins are readily processed using conventional extrusion, injection molding, compression molding, and vacuum forming techniques well known in the art. Useful articles made from them include films, fibers, bottles and other containers, sheeting, and molded objects.

Low molecular weight resins are useful, for example, as synthetic waxes and they may be used in various wax coatings or in emulsion form. They are also particularly useful in blends with ethylene/vinyl acetate or ethylene/methyl acrylate-type copolymers in paper coating or in adhesive applications.

Although not required, typical additives used in olefin or vinyl polymers may be used in the new homopolymers and copolymers of this invention. Typical additives include pigments, colorants, titanium dioxide, carbon black, antioxidants, stabilizers, slip agents, flame retarding agents, and the like. These additives and their use in polymer systems are known per se in the art.

The molecular weight data presented in the following examples is reported as linear poly(ethylene) equivalent molecular weights determined at 135 °C in 1,2,4-trichlorobenzene using refractive index detection, calibrated using narrow molecular weight distribution poly(styrene) standards, and employing the appropriate Mark-Houwink-Sakurada (MHS) rotations.

### EXAMPLES

Other features of the invention will become apparent in the course of the following descriptions of exemplary embodiments which are given for illustration of the invention,

### Example 1

### N,N'-Bis(2,6-dimethylphenyl)oxalamide

2,6-Dimethylaniline, triethylamine, and dichloromethane were dried by passage through basic alumina. A 1 L round bottom flask, equipped with a magnetic stir bar and a 125 mL pressure-equalizing dropping funnel capped by a nitrogen inlet adapter, was charged with 53.38 g of 2,6-dimethylaniline, 250 mL of dichloromethane, and 44.76 g of triethylamine. A solution of 25.34 g of oxalyl chloride in 80 mL of dichloromethane was added dropwise under nitrogen with stirring and ice-bath cooling over 1.2 hours to give a thick paste which had to be occasionally swirled by hand to effect mixing. The mixture was allowed to stir at room temperature for 14 hours, then transferred to a separatory funnel, washed 3 times with water, separated and concentrated under reduced pressure 1.3 kPa (10 mn Hg) to give 63 g of crude solid. The crude product was dissolved in a boiling mixture of 2850 mL of toluene and 1300 mL of absolute ethanol, cooled to room temperature and diluted with 260 mL of water, then allowed to crystallize for 16 hours. The resultant precipitate was isolated by vacuum filtration, washed with methanol (3 x 100 mL) and dried to give 39.1 g (66%) as white crystals. An additional 9.5 g (16.1%) was recovered from the filtrate by further dilution with ca. 500 mL water. Field desorption mass spectrometry showed a parent ion peak at 296 m/z. ¹H NMR (300 MHz, CDCl₃, chemical shifts in ppm relative to TMS at 0 ppm): 2.29 (12 p, s), 7.15 (6 p, m), 8.86 (2 p. br s).

### Example 2

### Preparation of N¹,N²-bis(2,6-dimethylphenyl)oxalodiimidoyl dichloride.

A 1 L round bottom flask was charged with 30.0 g of *N,N*'-bis(2,6-dimethylphenyl)oxalamide, 58.75 g of phosphorous pentachloride and 150 mL of dry toluene, and equipped with a magnetic stir bar and a reflux condenser capped by a nitrogen inlet adapter connected to a bubbler. The mixture was heated to reflux over 30 minutes, then maintained at reflux under nitrogen for another 95 minutes to give a yellow solution. Heating was discontinued and the mixture was allowed to cool to room temperature. A short path distillation adapter and receiving flask were attached in place of the condenser, and the volatiles were removed under reduced pressure (0.13 kPa (1 mm Hg)), initially at room temperature, then at 100°C, to give 20.1 g (60%) of a granular yellow solid. Field desorption mass spectrometry showed a parent ion peak at 332 m/z. ¹H NMR (300 MHz, C₆D₆, chemical shifts in ppm relative to TMS at 0 ppm): 2.04 (12 p, s), 6.91 (6 p, s).

### Example 3

### Preparation of (2,6-dimethylphenyl)-[5-(2,6-dimethylphenylimino)-2-phenyl-5H-imidazol-4-yl]amine (compound II with Q = CPh and R¹ = R² = 2,6-dimethylphenyl)

A 100 mL, round bottom flask equipped with a magnetic stir bar and condenser capped by a nitrogen inlet adapter was charged with 347 mg of benzamidine hydrochloride hydrate, 25 mL of dry N-methylpyrrolidinone and 1.47 g of triethylamine. The mixture was stirred for 5 minutes, then treated with 499 mg of *N*¹,*N*²-bis(2,6-dimethylphenyl)oxalodiimidoyl dichloride. A nitrogen atmosphere was established, then the mixture was heated to 85° C and maintained at that temperature for 6 days. The resultant dark red-brown mixture was diluted with ether and washed with water, and the ether layer was concentrated under reduced pressure (1.3 kPa (10 mm Hg, 85°C bath)) to remove most of the volatiles. Flash chromatography (SiO₂, 12 % EtOAc/ 88% hexanes) yielded 96 mg of an orange powder. Recrystallization from CH₂Cl₂/hexane gave 65 mg of dark violet crystals of the title compound. Field desorption mass spectrometry showed a parent ion peak at 380 m/z. ¹H NMR (500 MHz, CD₂Cl₂, chemical shifts in ppm relative to TMS at 0 ppm): 2.28 (12 p, s), 7.10-7.25 (6 p, s). 7.38-7.48 (2 p, m), 7.35-7.63 (1 p, m), 8.25-8.3 (2 p, m).

### Example 4

### Polyethylene Synthesis Using a Catalyst Generated In Situ from (2,6-dimethylphenyl)-[5-(2,6-dimethylphenylimino)-2-phenyl-5H-imidazol-4-yl]amine (compound II with Q = CPh and R¹ = R² = 2,6-dimethylphenyl) and Bis(1,5-cyclooctadiene)nickel(0)

In a drybox, a flame-dried, 200 mL, pear-shaped Schlenk flask equipped with a magnetic stir bar and capped by a septum was charged with 10 mg of (2,6-dimethylphenyl)-[5-(2,6-dimethylphenylimino)-2-phenyl-5*H*-imidazol-4-yl]amine and 5 mg of bis(1,5-cyclooctadiene)nickel(0). On the Schlenk line, the flask was evacuated, refilled with ethylene, then charged with 45 mL of dry, deoxygenated toluene, introduced via syringe, with stirring. A transient violet color was observed, which rapidly changed to a dark-green or red-green calor. Ethylene uptake was observed. After 2 hours of stirring under 0.13 kPa (1 atm) of ethylene at 21°C, the solution was golden-brown and methanol was added to precipitate the polymer as an oily gum. The volatiles were removed under reduced pressure (1.3 kPa initially, then 53 Pa (10 mm Hg initially, then 0.4 mm Hg)) to give 0.20g of a viscous beige polyethylene gum. GPC (relative to polystyrene standards) Mₙ = 38,000; M_{w} = 73,000. ¹H NMR (o-dichlorobenzene-d') showed ca. 112 branches per 1000 carbons.

### Example 5

### Polyethylene Synthesized Using a Catalyst Generated In Situ from (2,6-dimethylphenyl)-[5-(2,6-dimethylphenylimino)-2-phenyl-5H-imidazol-4-yl]amine (compound II with Q = CPh and R¹ = R² = 2,6-dimethylyhenyl, Bis(1,5-cyclooctadiene)nickel(0) and Tris(pentafluorophenyl)boron

In the drybox, a flame-dried, 500 mL, round bottom flask equipped with a magnetic stir bar was charged with 8 mg of (2,6-dimethylphenyl)-[5-(2,6-dimethylphenylimino)-2-phenyl-5*H*-imidazol-4-yl]amine, 3 mg of bis(1,5-cyclooctadiene)nickel(0) and 120 mg of tris(pentafluorophenyl)boron, then fitted with a side-arm adapter equipped with a Kontes high vacuum valve and a 24/40 septum. On the Schlenk line, the flask was evacuated, refilled with ethylene, then charged with 200 mL of dry, deoxygenated toluene, introduced via syringe, with stirring. A dark red solution resulted, which subsequently became somewhat more brown and slightly purple over the next 10-20 minutes. Ethylene uptake was observed. After 60 minutes of stirring under 1 atmosphere of ethylene at 23° C, methanol and acetone were added to produce an off-white supernatant and a white flocculent precipitate of polyethylene. The polymer was isolated by vacuum filtration and dried under reduced pressure (33 kPa (250 mm Hg) at 100°C for 14 h to afford 2.6 g of a white, polyethylene powder. GPC (relative to polystyrene standards) Mₙ = 8.200: M_{w} = 17,900. DSC: (2nd heat) melt transition with endothermic maxima at 78 and 91 °C. ¹H NMR (o-dichlorobenzene-d⁴) showed approximately 44 branches per 1000 carbons.

### Example 6

### Copolymer Synthesized From Ethylene and Propene Using a Catalyst Generated In Situ from (2,6-dimethylphenyl)-[5-(2,6-dimethylphenylimino)-2-phenyl-5H-imidazol-4-yl]amine (compound II with Q = CPh and R¹ = R² = 2,6-dimethylphenyl), Bis(1,5-cyclooctadiene)nickel(0) and Tris(pentafluorophenyl)boron

In the drybox, a flame-dried, 500 mL, round bottom flask equipped with a magnetic stir bar was charged with 10 mg of (2,6-dimethylphenyl)-[5-(2,6-dimethylphenylimino)-2-phenyl-5*H*-imidazol-4-yl]amine, 5 mg of bis(1,5-cyclooctadiene)nickel(0) and 106 mg of tris(pentafluorophenyl)boron, then fitted with an inert gas inlet adapter equipped with a Kontes high vacuum valve and a 24/40 septum. On the Schlenk line, the flask was evacuated, refilled with ethylene, then charged with 100 mL of dry, deoxygenated toluene, introduced via syringe, with stirring. A dark red solution resulted, which subsequently became somewhat more brown and slightly purple over the next 10 min, after which the ethylene was replaced by propene (133 Pa (1 atm)). After a total of 120 min, the reaction was quenched with methanol and worked up to obtain the 1.8 g of the copolymer as an oily gum. GPC (relative to polystyrene standards) Mₙ = 3,535; M_{w} = 10,353.). ¹H NMR (500 MHz, o-dichlorobenzene-d⁴) showed approximately 160 branches per 1000 carbons.

### Example 7

### Polyethylene Synthesized Using a Catalyst Generated In Situ from (2,6-dimethylphenyl)-[5-(2,6-dimethylphenylimino)-2-phenyl-5H-imidazol-4-vllamine (compound II with Q = CPh and R¹ = R² = 2,6-dimethylphenyl), Bis(1,5-cyclooctadiene)nickel(0) and Tris(pentafluorophenyl)boron

In the drybox, a flame-dried, 500 mL, round bottom flask equipped with a magnetic stir bar was charged with 10 mg of (2,6-dimethylphenyl)-[5-(2,6-dimethylphenyl)-2-phenyl-5*H*-imidazol-4-yl]amine, 5 mg of bis(1,5-cyclooctadiene)nickel(0) and 106 mg of tris(pentafluorophenyl)boron, then fitted with an inert gas inlet adapter equipped with a Kontes high vacuum valve and a 24/40 septum. On the Schlenk line, the flask was evacuated, refilled with ethylene, then charged with 100 mL of dry, deoxygenated toluene, introduced via syringe, with stirring. A dark red solution resulted, which subsequently became somewhat more brown and slightly purple over the next 10 min. After a total of 30 min stirring under an ethylene atmosphere, the reaction was quenched by the addition of methanol and acetone and the resultant precipitate was isolated by filtration and dried in vacuo to obtain 5.0 g of white, powdery polyethylene. GPC (relative to polystyrene standards) Mₙ = 10,500; M_{w} = 27.500. ¹H NMR (500 MHz, o-dichlorobenzene-d⁴) showed approximately 70 branches per 1000 carbons.

## Claims

1. A compound of formula **II:** 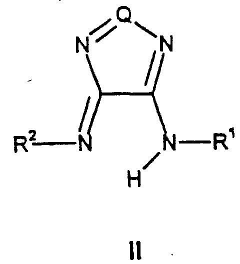 wherein R¹ and R² are both sterically hindered aryl rings;
Q is (i) C-R⁴, where R⁴ is hydrocarbyl, substituted hydrocarbyl, heteroatom connected hydrocarbyl, or heteroatom connected substituted hydrocarbyl, (ii) P(NH₂)₂, or (iii) S(NH) (NH₂) or S(O)(OH).

2. The compound of claim 1 further comprising a boron or aluminium containing neutral Lewis acid.

3. A batch or continuous process for the polymerization of olefins, comprising: contacting, at a temperature from -100°C to 200°C,one or more monomers of the formula RCH=CHR³, with (i) a compound of the formula **II** according to claim 1, or tautomers thereof, (ii) a suitable precursor selected from zerovalent Ni, Pd, Co, or Fe or compounds thereof, and, optionally, (iii) a neutral Lewis acid, wherein R and R³ are each independently hydrogen, hydrocarbyl or fluoroalkyl,and may be linked to form a cyclic olefin.

4. The process of claim 3 wherein the suitable precursor is a zerovalent Ni.

5. The process of claim 3 or claim 4 wherein the monomer is selected from ethylene, propylene, 1-butene, 1-pentene, 1-hexene, 1-octene or cyclopentene.

6. A batch or continuous process for the polymerization of olefins, comprising: contacting, at a temperature from -100°C to 200°C, one or more monomers of the formula RCH=CHR³, with (i)an anionic compound of the formula **III**, (ii) a suitable divalent metal precursor selected from Ni(II), Pd(II), Co(II), or Fe(II) compounds, and, optionally, (iii) a neutral Lewis acid; 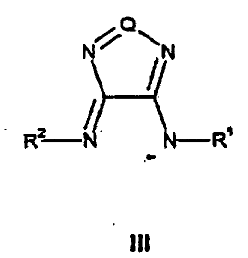 wherein R and R³ are each, independently, hydrogen, hydrocarbyl, or fluoroalkyl, and may be linked to form a cyclic olefin;
R¹ and R² are each independently hydrocarbyl, substituted hydrocarbyl, or silyl;
Q is C-R⁴, wherein R⁴ is hydrocarbyl, substituted hydrocarbyl, heteroatom connected hydrocarbyl, heteroatom connected substituted hydrocarbyl, or O-Si(tert-butyl)(CH₃)₂.

7. The process of claim 6 wherein the suitable divalent metal precursor is a Ni(II) compound.

8. The process of claim 6 or claim 7 wherein Q is C-R⁴, where R⁴ is hydrocarbyl, or substituted hydrocarbyl.

9. The process of claim 6 or claim 7 wherein Q is C-R⁴, where R⁴ is phenyl, pentafluorophenyl, trifluoromethyl, fluoroalkyl, O-Si(tert-butyl)(CH₃)₂, or SCH₃.

10. The process of any of claims 6 to 9 wherein the monomer is selected from ethylene, propylene, 1-butene, 1-pentene, 1-hexene, 1-octene or cyclopentene.

11. A compound of formula **I:** 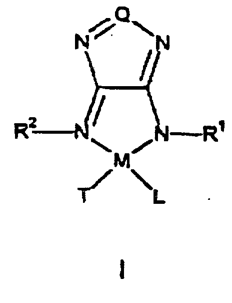 wherein R¹ and R² are each independently hydrocarbyl, substituted hydrocarbyl, or silyl;
Q is (i) C-R⁴, where R⁴ is hydrocarbyl, substituted hydrocarbyl, heteroatom connected hydrocarbyl, or heteroatom connected substituted hydrocarbyl, (ii) P(NH₂)₂, or (iii) S(NH)(NH₂) or S(O)(OH);
L is a monoolefin or a neutral Lewis base that can be displaced by a monoolefin;
T is hydrogen, hydrocarbyl or substituted hydrocarbyl, or may be taken together with L to form a π-allyl group; and
M is Ni(II), Pd(II), Co(II) or Fe(II).

12. The compound of claim 11 wherein M is nickel(II).

13. The compound of claim 11 or claim 12 wherein R¹ and R² are both sterically hindered aryl rings.

14. The compound of any of claims 11 to 13 further comprising a boron or aluminium containing neutral Lewis acid.

15. A batch or continuous process for the production of polyolefins, comprising: contacting, at a temperature from about -100°C to 200°C, one or more monomers of the formula RCH=CHR³, with (i) a transition metal compound of formula **I** according to any of claims 11 to 14, and, optionally, (ii) a Lewis acid: wherein R and R³ are each, independently, hydrogen, hydrocarbyl, or substituted hydrocarbyl such as fluoroalkyl, and may be linked to form a cyclic olefin.

16. The process of claim 15 wherein the monomer is selected from ethylene, propylene, 1-butene, 1-pentene, 1-hexene, or 1-octene.

17. The compound or process of claim 1 or claim 3
wherein R⁴ is phenyl, 4-methoxyphenyl, 4-cyanophenyl, trifluoromethyl, 4-nitrophenyl, or pentafluorophenyl.

18. A batch or continuous process for the polymerization of olefins, comprising: contacting one or more monomers of the formula RCH=CHR³ with a binucleating or multinucleating ligand complexed to a Group 8-10 transition metal M and one or more Lewis acids, wherein the Lewis acid or acids are bound to one or more heteroatoms which are π-conjugated to the donor atom or atoms bound to the transition metal M; and wherein R and R³ each, independently, represent a hydrogen, a hydrocarbyl, a fluoroalkyl, or may be linked to form a cyclic olefin.

19. The process of claim 18 wherein M is Ni(II).

20. The process of claim 18 or claim 19 wherein the binucleating or multinucleating ligand is a compound of formula II according to claim 1.

21. A composition comprising (i) a Group 8-10 transition metal M, (ii) one or more Lewis acids, and (iii) a binucleating or multinucleating compound of formula **II** according to claim 1;
wherein the Lewis acid or acids are bound to one or more heteroatoms which are π-conjugated to the donor atom or atoms bound to the transition metal M.

22. The composition of claim 21 wherein the transition metal M is Ni(II), the Lewis acid is a boron or aluminium containing Lewis acid, and Q is C-R⁴, where R⁴ is hydrocarbyl, substituted hydrocarbyl, heteroatom connected hydrocarbyl, or heteroatom connected substituted hydrocarbyl.

23. The compound or process of any preceding claim wherein Q is C-R⁴, where R⁴ is hydrocarbyl, substituted hydrocarbyl, heteroatom connected hyrocarbyl, or heteroatom connected substituted hydrocarbyl.

24. The compound or composition of any of claims 1, 11, 12, 13, 21, 23 further comprising a neutral Lewis acid.

25. The process of any of claims 3 to 10 or 15 to 20 wherein a neutral Lewis acid is utilized.

## Patentansprüche

1. Eine Verbindung der Formel II: 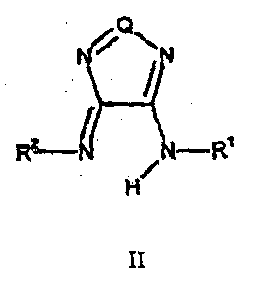 worin R¹ und R² beide sterisch gehinderte Arylringe sind;
Q (i) C-R⁴, wobei R⁴ Hydrocarbyl, substituiertes Hydrocarbyl, Heteroatom-verbundenes Hydrocarbyl oder substituiertes Heteroatom-verbundenes Hydrocarbyl ist, (ii) P(NH₂)₂ oder (iii) S(NH) (NH₂) oder S(O) (OH) ist.

2. Die Verbindung nach Anspruch 1, welche weiterhin eine Bor oder Aluminium enthaltende neutrale Lewis-Säure umfasst.

3. Ein diskontinuierliches oder kontinuierliches Verfahren zur Polymerisation von Olefinen, umfassend: Inkontaktbringen bei einer Temperatur von -100°C bis 200°C eines oder mehrerer Monomere der Formel RCH=CHR³ mit (i) einer Verbindung der Formel II gemäß Anspruch 1 oder Tautomeren von dieser, (ii) einem geeigneten Vorläufer, der ausgewählt wird aus nullwertigem Ni, Pd, Co oder Fe oder Verbindungen von diesen, und gegebenenfalls (iii) einer neutralen Lewis-Säure, worin R und R³ jeweils unabhängig Wasserstoff, Hydrocarbyl oder Fluoralkyl sind und verknüpft sein können, um ein cyclisches Olefin zu bilden.

4. Das Verfahren nach Anspruch 3, wobei der geeignete Vorläufer ein nullwertiges Ni ist.

5. Das Verfahren nach Anspruch 3 oder Anspruch 4, wobei das Monomer ausgewählt wird aus Ethylen, Propylen, 1-Buten, 1-Penten, 1-Hexen, 1-Octen oder Cyclopenten.

6. Ein diskontinuierliches oder kontinuierliches Verfahren zur Polymerisation von Olefinen, umfassend: Inkontaktbringen bei einer Temperatur von -100°C bis 200°C eines oder mehrerer Monomere der Formel RCH=CHR³ mit (i) einer anionischen Verbindung der Formel III, (ii) einem geeigneten zweiwertigen Metallvorläufer, der ausgewählt wird aus Ni(II)-, Pd(II)-, Co(II)- oder Fe(II)-Verbindungen, und gegebenenfalls (iii) einer neutralen Lewis-Säure; 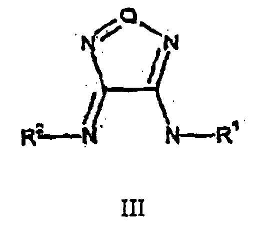 worin R und R³ jeweils unabhängig Wasserstoff, Hydrocarbyl oder Fluoralkyl sind und verknüpft sein können, um ein cyclisches Olefin zu bilden;
R¹ und R² jeweils unabhängig Hydrocarbyl, substituiertes Hydrocarbyl oder Silyl sind;
Q C-R⁴ ist, wobei R⁴ Hydrocarbyl, substituiertes Hydrocarbyl, Heteroatom-verbundenes Hydrocarbyl oder substituiertes Heteroatom-verbundenes Hydrocarbyl oder O-Si(tert.-Butyl)(CH₃)₂ ist.

7. Das Verfahren nach Anspruch 6, wobei der geeignete zweiwertige Metallvorläufer eine Ni(II)-Verbindung ist.

8. Das Verfahren nach Anspruch 6 oder Anspruch 7, wobei Q C-R⁴ ist, wobei R⁴ Hydrocarbyl oder substituiertes Hydrocarbyl ist.

9. Das Verfahren nach Anspruch 6 oder Anspruch 7, wobei Q C-R⁴ ist, wobei R⁴ Phenyl, Pentafluorphenyl, Trifluormethyl, Fluoralkyl, O-Si(tert.-Butyl)(CH₃)₂ oder SCH₃ ist.

10. Das Verfahren nach irgendeinem der Ansprüche 6 bis 9, wobei das Monomer ausgewählt wird aus Ethylen, Propylen, 1-Buten, 1-Penten, 1-Hexen, 1-Octen oder Cyclopenten,

11. Eine Verbindung der Formel I: 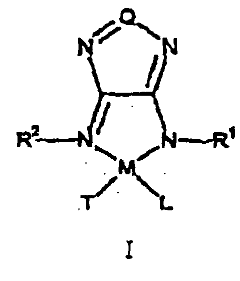 worin R¹ und R² jeweils unabhängig Hydrocarbyl, substituiertes Hydrocarbyl oder Silyl sind;
Q (i) C-R⁴, wobei R⁴ Hydrocarbyl, substituiertes Hydrocarbyl, Heteroatom-verbundenes Hydrocarbyl oder substituiertes Heteroatom-verbundenes Hydrocarbyl ist, (ii) P(NH₂)₂ oder (iii) S(NH) (NH₂) oder S(O) (OH) ist;
L ein Monoolefin oder eine neutrale Lewis-Base ist, die durch ein Monoolefin ersetzt werden kann;
T Wasserstoff, Hydrocarbyl oder substituiertes Hydrocarbyl ist oder mit L zusammen genommen werden kann, um eine π-Allylgruppe zu bilden; und
M Ni(II), Pd(II), Co(II) oder Fe(II) ist.

12. Der Verbindung nach Anspruch 11, wobei M Nickel(II) ist.

13. Der Verbindung nach Anspruch 11 oder Anspruch 12, wobei R¹ und R² beide sterisch gehinderte Arylringe sind.

14. Die Verbindung nach irgendeinem der Ansprüche 11 bis 13, welche weiterhin eine Bor oder Aluminium enthaltende neutrale Lewis-Säure umfasst.

15. Ein diskontinuierliches oder kontinuierliches Verfahren zur Herstellung von Polyolefinen, umfassend: Inkontaktbringen bei einer Temperatur von ca. -100°C bis 200°C eines oder mehrerer Monomere der Formel RCH=CHR³ mit (i) einer Übergangsmetallverbindung der Formel I gemäß irgendeinem der Ansprüche 11 bis 14 und gegebenenfalls (ii) einer Lewis-Säure; wobei R und R³ jeweils unabhängig Wasserstoff, Hydrocarbyl oder substituiertes Hydrocarbyl wie z.B. Fluoralkyl sind und verknüpft sein können, um ein cydisches Olefin zu bilden.

16. Das Verfahren nach Anspruch 15, wobei das Monomer ausgewählt wird aus Ethylen, Propylen, 1-Buten, 1-Penten, 1-Hexen oder 1-Octen.

17. Die Verbindung oder das Verfahren nach Anspruch 1 oder Anspruch 3, wobei R⁴ Phenyl, 4-Methoxyphenyl, 4-Cyanophenyl, Trifluormethyl, 4-Nitrophenyl oder Pentafluorphenyl ist.

18. Ein diskontinuierliches oder kontinuierliches Verfahren zur Polymerisation von Olefinen, umfassend: Inkontaktbringen eines oder mehrerer Monomere der Formel RCH=CHR³ mit einem zweikemigen oder mehrkemigen Liganden, der mit einem Übergangsmetall M der Gruppe 8 - 10 und einer oder mehreren Lewis-Säuren komplexiert ist, wobei die Lewis-Säure oder -Säuren an ein oder mehrere Heteroatome gebunden ist/sind, welche mit dem Donoratom oder den Donoratomen, die an das Übergangsmetall M gebunden sind, π-konjugiert sind; und wobei R und R³ jeweils unabhängig für einen Wasserstoff, ein Hydrocarbyl, ein Fluoralkyl stehen oder verknüpft sein können, um ein cyclisches Olefin zu bilden.

19. Das Verfahren nach Anspruch 18, wobei M Ni(II) ist.

20. Das Verfahren nach Anspruch 18 oder Anspruch 19, wobei der zweikernige oder mehrkernige Ligand eine Verbindung der Formel II gemäß Anspruch 1 ist.

21. Eine Zusammensetzung, umfassend: (i) ein Übergangsmetall M der Gruppe 8 - 10, (ii) eine oder mehrere Lewis-Säuren und (iii) eine zweikernige oder mehrkernige Verbindung der Formel II gemäß Anspruch 1;
wobei die Lewis-Säure oder -Säuren an ein oder mehrere Heteroatome gebunden ist/sind, welche mit dem Donoratom oder den Donoratomen, die an das Übergangsmetall M gebunden sind, n-konjugiert sind.

22. Die Zusammensetzung nach Anspruch 21, wobei das Übergangsmetall M Ni(II) ist, die Lewis-Säure eine Bor oder Aluminium enthaltende Lewis-Säure ist, und Q C-R⁴ ist, wobei R⁴ Hydrocarbyl, substituiertes Hydrocarbyl, Heteroatom-verbundenes Hydrocarbyl oder substituiertes Heteroatom-verbundenes Hydrocarbyl ist.

23. Die Verbindung oder das Verfahren nach irgendeinem vorhergehenden Anspruch, wobei Q C-R⁴ ist, wobei R⁴ Hydrocarbyl, substituiertes Hydrocarbyl, Heteroatom-verbundenes Hydrocarbyl oder substituiertes Heteroatom-verbundenes Hydrocarbyl ist.

24. Die Verbindung oder Zusammensetzung nach irgendeinem der Ansprüche 1, 11, 12, 13, 21, 23, welche weiterhin eine neutrale Lewis-Säure umfasst.

25. Das Verfahren nach irgendeinem der Ansprüche 3 bis 10 oder 15 bis 20, wobei eine neutrale Lewis-Säure benutzt wird.

## Revendications

1. Composé de formule II : 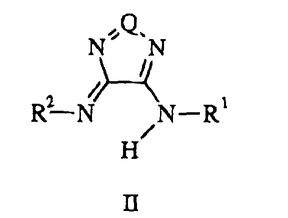 dans laquelle R¹ et R² sont tous deux des cycles aryle stériquement encombrés ;
Q est (i) un groupe C-R⁴, dans lequel R⁴ est un groupe hydrocarbyle, hydrocarbyle substitué, hydrocarbyle relié par un hétéroatome ou hydrocarbyle substitué relié par un hétéroatome, (ü) un groupe P(NH₂)₂ ou (iii) un groupe S(NH)(NH₂) ou S(O)(OH).

2. Composé selon la revendication 1 comprenant en outre un acide de Lewis neutre contenant du bore ou de l'aluminium.

3. Procédé discontinu ou continu pour la polymérisation des oléfines comprenant : la mise en contact, à une température de -100°C à 200°C, d'un ou plusieurs monomères de formule RCH=CHR³ avec (i) un composé de formule II selon la revendication 1 ou un de ses tautomères, (ü) un précurseur approprié choisi parmi Ni, Pd, Co ou Fe de valence nulle ou un de leurs composés et éventuellement (iii) un acide de Lewis neutre, dans laquelle R et R³ sont chacun indépendamment un atome d'hydrogène, un groupe hydrocarbyle ou fluoroalkyle et ils peuvent être reliés pour former une oléfine cyclique.

4. Procédé selon la revendication 3, dans lequel le précurseur approprié est Ni de valence nulle.

5. Procédé selon la revendication 3 ou la revendication 4, dans lequel le monomère est choisi parmi l'éthylène, le propylène, le 1-butène, le 1-pentène, le 1-hexène, le 1-octène ou le cyclopentène.

6. Procédé discontinu ou continu pour la polymérisation des oléfines comprenant : la mise en contact, à une température de -100°C à 200°C, d'un ou plusieurs monomères de formule RCH=CHR³ avec (i) un composé anionique de formule III, (ü) un précurseur métallique divalent approprié choisi parmi les composés de Ni(II), Pd(II), Co(II) ou Fe(II) et éventuellement (iii) un acide de Lewis neutre ; 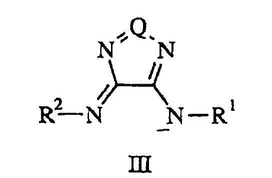 dans laquelle R et R³ sont chacun indépendamment un atome d'hydrogène, un groupe hydrocarbyle ou fluoroalkyle et ils peuvent être reliés pour former une oléfine cyclique ;
R¹ et R² sont chacun indépendamment un groupe hydrocarbyle, hydrocarbyle substitué ou silyle ;
Q est un groupe C-R⁴, dans lequel R⁴ est un groupe hydrocarbyle, hydrocarbyle substitué, hydrocarbyle relié par un hétéroatome ou hydrocarbyle substitué relié par un hétéroatome ou un groupe O-Si(tert-butyle)(CH₃)₂.

7. Procédé selon la revendication 6, dans lequel le précurseur métallique divalent approprié est un composé de Ni(II).

8. Procédé selon la revendication 6 ou la revendication 7, dans lequel Q est un groupe C-R⁴, dans lequel R⁴ est un groupe hydrocarbyle ou hydrocarbyle substitué.

9. Procédé selon la revendication 6 ou la revendication 7, dans lequel Q est un groupe C-R⁴, dans lequel R⁴ est un groupe phényle, pentafluorophényle, trifluorométhyle, fluoroalkyle, O-Si(tert-butyle)(CH₃)₂ ou SCH₃.

10. Procédé selon l'une quelconque des revendications 6 à 9, dans lequel le monomère est choisi parmi l'éthylène, le propylène, le 1-butène, le 1-pentène, le 1-hexène, le 1-octène ou le cyclopentène.

11. Composé de formule I : 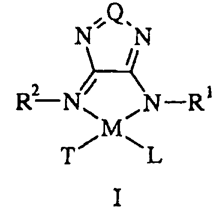 dans laquelle R¹ et R² sont chacun indépendamment un groupe hydrocarbyle, hydrocarbyle substitué ou silyle ;
Q est (i) un groupe C-R⁴, dans lequel R⁴ est un groupe hydrocarbyle, hydrocarbyle substitué, hydrocarbyle relié par un hétéroatome ou hydrocarbyle substitué relié par un hétéroatome, (ii) un groupe P(NH₂)₂ ou (iii) un groupe S(NH)(NH₂) ou S(O)(OH) ;
L est une monooléfine ou une base de Lewis neutre qui peut être déplacée par une monooléfine ;
T est un atome d'hydrogène, un groupe hydrocarbyle ou hydrocarbyle substitué ou il peut être pris ensemble avec L afin de former un groupe π-allyle ; et
M est Ni(II), Pd(II), Co(II) ou Fe(II).

12. Composé selon la revendication 11, dans lequel M est le nickel(II).

13. Composé selon la revendication 11 ou la revendication 12, dans lequel R¹ et R² sont tous deux des cycles aryle stériquement encombrés.

14. Composé selon l'une quelconque des revendications 11 à 13, comprenant en outre un acide de Lewis neutre contenant du bore ou de l'aluminium.

15. Procédé discontinu ou continu pour la production de polyoléfines comprenant : la mise en contact, à une température d'environ -100°C à 200°C, d'un ou plusieurs monomères de formule RCH=CHR³ avec (i) un composé d'un métal de transition de formule I selon l'une quelconque des revendications 11 à 14 et éventuellement (ii) un acide de Lewis : dans lequel R et R³ sont chacun indépendamment un atome d'hydrogène, un groupe hydrocarbyle ou hydrocarbyle substitué, tel qu'un groupe fluoroalkyle, et ils peuvent être reliés pour former une oléfine cyclique.

16. Procédé selon la revendication 15, dans lequel le monomère est choisi parmi l'éthylène, le propylène, le 1-butène, le 1-pentène, le 1-hexène ou le 1-octène.

17. Composé ou procédé selon la revendication 1 ou la revendication 3, dans lequel R⁴ est un groupe phényle, 4-méthoxyphényle, 4-cyanophényle, trifluorométhyle, 4-nitrophényle ou pentafluorophényle.

18. Procédé discontinu ou continu pour la polymérisation des oléfines comprenant : la mise en contact d'un ou plusieurs monomères de formule RCH=CHR³ avec un ligand de bicoordination ou de multicoordination complexé à un métal de transition M des groupes 8 à 10 et un ou plusieurs acides de Lewis, dans lequel l'acide ou les acides de Lewis est(sont) lié(s) à un ou plusieurs hétéroatomes qui sont conjugués en π à l'atome donneur ou aux atomes donneurs lié(s) au métal de transition M ; et dans lequel R et R³ représentent chacun indépendamment un atome d'hydrogène, un groupe hydrocarbyle, un groupe fluoroalkyle ou ils peuvent être reliés pour former une oléfine cyclique.

19. Procédé selon la revendication 18 dans lequel M est Ni(II).

20. Procédé selon la revendication 18 ou la revendication 19, dans lequel le ligand de bicoordination ou de multicoordination est un composé de formule II selon la revendication 1.

21. Composition comprenant (i) un métal de transition M des groupes 8 à 10, (ii) un ou plusieurs acides de Lewis et (iii) un composé de bicoordination ou de multicoordination de formule II selon la revendication 1 ;
dans laquelle l'acide de Lewis ou les acides de Lewis est(sont) lié(s) à un ou plusieurs hétéroatomes qui sont conjugués en π à l'atome donneur ou aux atomes donneurs lié(s) au métal de transition M.

22. Composition selon la revendication 21, dans laquelle le métal de transition M est Ni(II), l'acide de Lewis est un acide de Lewis contenant du bore ou de l'aluminium et Q est un groupe C-R⁴, dans lequel R⁴ est un groupe hydrocarbyle, hydrocarbyle substitué, hydrocarbyle relié par un hétéroatome ou hydrocarbyle substitué relié par un hétéroatome.

23. Composé ou procédé selon l'une quelconque des revendications précédentes dans lequel Q est un groupe C-R⁴, dans lequel R⁴ est un groupe hydrocarbyle, hydrocarbyle substitué, hydrocarbyle relié par un hétéroatome ou hydrocarbyle substitué relié par un hétéroatome.

24. Composé ou composition selon l'une quelconque des revendications 1, 11, 12, 13, 21, 23 comprenant en outre un acide de Lewis neutre.

25. Procédé selon l'une quelconque des revendications 3 à 10 ou 15 à 20, dans lequel on utilise un acide de Lewis neutre.
